# EUROPEAN PATENT APPLICATION

(11) **EP 3 323 289 A1**
(43) Date of publication of application: **23.05.2018**
(21) Application number: 16199093.2
(22) Date of filing: 16.11.2016
(51) Int. Cl.: A01N 1/02

(54) **METHOD AND APPARATUS FOR IMPROVING THE QUALITY OF MAMMALIAN SPERM**

(71) Applicant: Instruments Utils de Laboratori Geniul, SL, 08030 Barcelona (ES)
(72) Inventor: CODONY IGLESIAS, Francesc, 08302 Mataró (ES); BONET MARULL, Sergi, 17003 Girona (ES); YESTE OLIVERAS, Marc, 17003 Girona (ES); RODRÍGUEZ GIL, Juan Enrique, 08271 Artés (ES)
(74) Representative: Juncosa Miro, Jaime

(57) **Abstract**

The method comprises irradiating said sperm cells with non-coherent red light, the irradiation with non-coherent red light being performed in a discontinuous manner according to a pattern including at least one sequence of two irradiation periods of specific durations which are separated by an intermediate period of darkness of a specific duration, wherein the sperm cells are diluted in a diluent with cryo-preservatives solutes and are included in said container in a frozen state, and said discontinuous pattern comprise a duration between 3 and 15 minutes.

## Description

### Field of the Invention

The present invention generally relates to mammal sperm treatment methods and apparatuses. In particular the invention relates to a method and to an apparatus for improving the quality of mammalian sperm (seminal fluid) to increase (tax of) fertility of the sperm, by means of, but not limited to, activating the motility of the spermatozoa, their resistance to stress and generally improving spermatozoa viability.

### Background of the Invention

Current modern farm practices often need the use of artificial insemination (Al) in order to optimize the reproductive cycles, select the sex of the animals or improving animal genetics using specific sperm lines. Especially in ungulates as ruminants (cow) and equines (mare), the standard practices are based in the use on frozen straws containing the diluted sperm solution along with cryo-preservatives solutes.

It is well known that the method of cryopreservation by itself or by the previous spermatozoa manipulation such the procedures used for spermatozoa selection-separation (e.g. need for selective production of calf females), can compromise the cell quality and the standard functional parameters in seminal fluids. Despite the loss of quality and their impact in the fertility rate, Al procedures still offers more practical operational advantages than the use of stud bull or stallion for natural insemination. However, Al in ungulates, (i.e. ruminants as cows or equines as mares) if compared with sows, showed lower % of pregnancy.

In the same context, for example in pig farming, the use of photoestimulation in commercial seminal doses stored at 17ºC (non-frozen) has been proved as successful methodology for improving quality parameters of seminal suspensions and for increasing the fertility rate in reproductive farms [1]. EP-A1-2997823 patent application, of the same inventors of present invention, is related with this work, in which a procedure for mammalian refrigerated diluted or non-diluted semen portions is disclosed. These procedures need large lighting cycles which are not compatible with the procedures based on stud bull or stallion frozen samples, which need fast thawing and quick use of one the sample at physiological temperature (35-40ºC). Otherwise, since the standard container format for frozen seminal samples in the veterinary field are small, thin and long containers (straws), containing low sperm volumes (< 1000 µl), there is necessary a specific instruments with a lighting geometry adapted to this container type.

There exist at least two molecular targets in sperm cells that could be activated by specifics wavelengths. One of them are the Opsins, placed in cellular membrane, and the other one are the mitochondrial electron transport chain which has been shown to be photosensitive. Recent works demonstrated that mammalian sperm cells, has happens in other cells from other tissues, contains a rhodopsin like proteins named Opsins that by the means of G-protein coupled receptors induces changes on cell cytoplasm. It has been hypothesized that in light absence, this proteins are involved in sperm thermotaxis [2]. On the other hand the mitochondrial electron transport chain has been shown to be photosensitive to red light, which activates ATP production and also affects the redox signalling that can be related with the homeostasis control [3].

Present invention involves an improvement of the teachings of cited EP-A1-2997823 patent application.

### Description of the Invention

Embodiments of the present invention provide in accordance with a first aspect a method for improving the quality of mammalian sperm, for instance to increase fertility of the sperm, said method comprising as known by the previous patent application EP-A1-2997823 irradiating sperm cells with non-coherent red light in a discontinuous manner according to a pattern including at least one sequence of two periods of sperm cell irradiation of a specific duration which are separated by an intermediate period of darkness of a specific duration. The sperm cells are included in a container having walls transparent to (allowing the passage of) the wavelength of said irradiated red light.

Unlike the known proposals in the field, in the proposed method the sperm cells are first diluted in a diluent with cryo-preservative solutes, thereby being at least in part diluted. Then, the sperm cells are included in said container and refrigerated (until reaching a frozen state). Finally, the sperm cells within the container are subjected to said discontinuous pattern of non-coherent red light that lasts between 3 and 15 minutes. Preferably the container is a straw suitable for keeping a volume of sperm cells of about 120µl to 250µl.

The sperm cells are mammalian sperm cells such ungulates (as ruminant and/or equine) sperm cells.

Preferably, the duration of said two periods of irradiation and of the intermediate period of darkness is the same. However said duration of the two periods of irradiation and of the intermediate period of darkness can also be different, always maintaining the duration of the complete discontinuous pattern of non-coherent red light.

Each of said specific periods of irradiation and darkness may have duration between 1 and 5 minutes. Preferably, each of said specific periods of irradiation and darkness has duration of 2 minutes. For instance, of 2 minutes for each period, i.e. 2 minutes irradiation, 2 minutes darkness and 2 minutes irradiation.

The non-coherent red light used by the proposed method may have a wavelength comprised in a range between 620 and 640 nm.

According to an embodiment, the diluted sperm cells contained in the container will be stored (<-35ºC) until their use. Once removed from the freezer, said discontinuous pattern of non-coherent red light is applied on at least one portion of said previously diluted but frozen semen sample (sperm cells). The diluted but frozen semen sample may be kept at least until a period of time immediately before the application of the discontinuous pattern of non-coherent red light thereon and/or during said application.

Moreover, the diluted but frozen semen sample, after being subjected to the discontinuous pattern of non-coherent red light, may be incubated in a heat controlled medium, for instance, at substantially near to animal physiological temperature up to 35-40ºC. The incubation can be performed for different periods of time. After each one of said different periods of time the method, according to an embodiment, performs analytical controls on the portion of the semen sample for determining at least a state of functional viability and motility of the spermatozoa.

Embodiments of the present invention provide in accordance with a second aspect an apparatus for improving the quality of mammalian sperm. The apparatus includes:
- non-coherent red light illumination means configured and arranged for irradiating sperm cells with non-coherent red light;
- control means configured for controlling said illumination means for performing said irradiation according to a discontinuous pattern including at least one sequence of two periods of sperm cell irradiation of a specific duration which are separated by an intermediate period of darkness of a specific duration;
- a support supporting at least one container such as a straw for containing said sperm cells, and the walls of which are transparent to the wavelength of said irradiated red light; and
- a casing for internally housing said support, the at least one container and the non-coherent red light illumination means.

In the proposed system, the sperm cells are preferably diluted in a diluent with cryo-preservative solutes and kept in the container in a frozen state, and the control means are configured to perform said discontinuous pattern of non-coherent red light between 3 and 15 minutes.

According to an embodiment, the system also includes overheating control means arranged and configured to ventilate the elements housed inside said casing, preferably avoiding temperatures greater than 40ºC during irradiation.

Therefore, present invention is based on treatment by means of exposing standard containers such as straws containing the seminal fluid to an electromagnetic (visible light) radiation. The treatment conditions have been optimized to specific wavelength, time and dose values in an isothermal environment.

The invention can be encompassed in the field of assisted reproductive techniques in mammals using frozen doses.

All the variables of exposure of the proposed method have been verified and optimized and the designed apparatus has been optimized for carrying out the proposed method on frozen containers (preferably straws) containing seminal suspensions, used in the steps prior to Al.

### Brief Description of the Drawings

The foregoing and other advantages and features will be better understood based on the following detailed description of several embodiments in reference to the attached drawings which must be interpreted in an illustrative and non-limiting manner, in which:
Fig. 1 a to Fig. 1 c illustrates different views of an embodiment of the proposed apparatus for implementing a method for improving the quality of mammalian sperm; and
Fig. 2 illustrates the electrical parts of the apparatus of Fig. 1.

### Detailed Description of several Embodiments

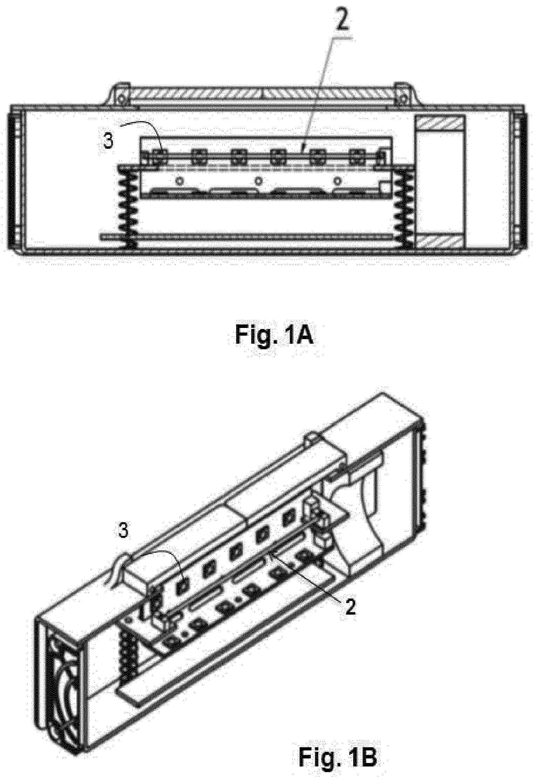

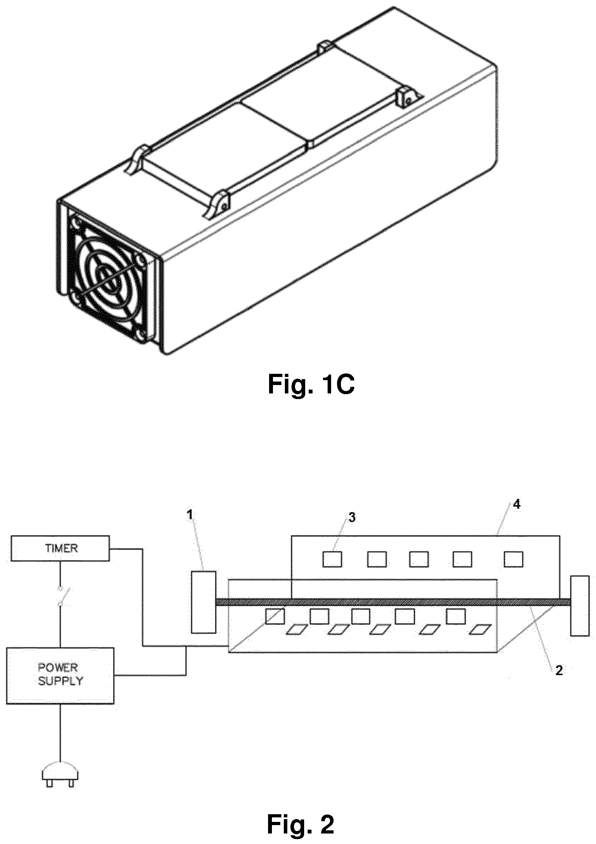

Present invention provides a method for improving the quality of mammalian sperm, in which diluted sperm cells, included in a container 2 (see Figs. 1 and 2) and refrigerated until reaching a frozen state (cryopreserved state) are irradiated with non-coherent red light of a wavelength between 620 and 640 nm in a discontinuous manner according to a pattern including at least one sequence of two periods of sperm cell irradiation of a specific duration which are separated by an intermediate period of darkness of a specific duration. The discontinuous pattern of non-coherent red light may last up to 15 minutes.

According to the invention, each of the specific periods of irradiation and darkness can have duration between 1 and 5 minutes. In an embodiment, the duration of each specific period is 1 minute (or even a little bit less). In another embodiment, the duration of each specific period is 5 minutes. Preferably, the duration of each specific period is 2 minutes.

The mentioned periods of exposure and darkness can have different duration.

In addition, the volume of the diluted sperm cells comprised in each container 2, preferably a straw having a size suitable for insemination treatment, may be comprised in a range between 120µl to 250µl.

To confirm the feasibility of phototherapy impact for improving the different descriptive parameters of sperm cell motility-viability, different laboratory studies were conducted using frozen Bull semen in straw, as a ruminant model.

Work as performed with a total of 20 cryopreserved semen samples coming from separated bulls. The samples were obtained and cryopreserved, according standard procedures [4], in straws of 0.25 mL and a final concentration of 92x10⁶ spermatozoa per mL. Different samples were exposed to a photoactivation instrument using LED emitting at 620-640 nm. Each sample was illuminated by three non-coherent red light illuminating means 3 (see Fig. 2) such as LEDs (two lateral and one at the bottom) at different intervals of time. Also non-irradiated samples were used as controls. The following table shows the results that were obtained.

| Treatment | Light (min) | Darkness (min) | Light (min) |
|---|---|---|---|
| Control | 0 | 0 | 0 |
| 1 * | 10 | 10 | 10 |
| 2 | 5 | 5 | 5 |
| 3 | 3 | 1 | 3 |
| 4 | 2 | 1 | 2 |
| 5 | 1 | 1 | 1 |

| | | | |
|---|---|---|---|
| * Light used in EP-A1-2997823 for non-frozen sperm (commercial boar ejaculates) | | | |

In each treatment the analytical tests were:
1. Evaluation of sperm viability: Flow cytometer procedure [5] adapted to bull sperm [6]. Briefly, sperm were incubated with 10nM SYBR-14 for 10 min at 37ºC in the dark, and then with 12 uM Propidium Iodide (PI) for 5 min at 37. Three replicates of each sample were evaluated, and 10.000 events were evaluated in each assessment.
2. Evaluation of sperm motility: using computer-assisted sperm analysis (CASA). Determinations were made using a Makler chamber under phase-contrast microscope at 10x magnifications. Three replicates of each sample (at least 1.000 sperm each) were evaluated and several kinetic parameters were determined, including straight-line velocity, average-path velocity, percentage of linearity, and percentages of total and progressively motile spermatozoa. Cut-off values were specific for bull sperm [6].

Results were analysed using a statistical software package. Experiments 1 and 2 were evaluated separately. In both cases, data were first checked for normality and homoscedasticity through Shapiro-Wilk and Levene tests. When required, data were transformed through arcsin (√x) prior to run a repeated measures ANOVA in which the inter-subject factor was the treatment (i.e. control vs. photo-stimulated) and the intra-subject factor was the time during which samples were kept at room temperature. Friedman and Wilcoxon tests were used when, even having been corrected, data did not match with parametric assumptions. Level of significance (*) was set at 5% and results are shown as mean ± standard error of the mean (SEM).

**Viability percentage (mean ± sem)**

| Treatment | Time (h) | % |
|---|---|---|
| Control | 0 | 51,0 ± 2,1 |
| | 2 | 45,0 ± 2,0 |
| | 4 | 43,1 ± 1,8 |
| | 24 | 39,4 ± 1,5 |
| #1 | 0 | 54,3 ± 2,5 |
| | 2 | 51,6 ± 2,2* |
| | 4 | 52,7 ± 2,4* |
| | 24 | 42,1 ± 2,0 |
| #2 | 0 | 50,3 ± 2,3 |
| | 2 | 52,1 ± 2,5* |
| | 4 | 49,3 ± 2,1* |
| | 24 | 41,5 ± 1,6 |
| #3 | 0 | 51,5 ± 2,5 |
| | 2 | 50,2 ± 2,1* |
| | 4 | 47,3 ± 2,2 |
| | 24 | 44,2 ± 1,9* |
| #4 | 0 | 52,1 ± 2,3 |
| | 2 | 50,3 ± 2,1* |
| | 4 | 49,9 ± 2,0* |
| | 24 | 42,4 ± 1,8 |
| #5 | 0 | 53,0 ± 2,4 |
| | 2 | 52,5 ± 2,3* |
| | 4 | 50,3 ± 1,9* |
| | 24 | 43,5 ± 2,0* |

**Motility percentage (mean ± sem)**

| Treatment | Time (h) | % |
|---|---|---|
| Control | 0 | 12,3 ± 0,9 |
| | 2 | 9,1 ± 1,2 |
| | 4 | 7,8 ± 1,4 |
| | 24 | 7,1 ± 1,1 |
| #1 | 0 | 16,3 ± 1,0* |
| | 2 | 16,5 ± 1,3* |
| | 4 | 14,1 ± 1,1* |
| | 24 | 12,2 ± 1,0* |
| #2 | 0 | 17,8 ± 1,0* |
| | 2 | 16,2 ± 1,2* |
| | 4 | 13,0 ± 0,9* |
| | 24 | 10,3 ± 0,9 |
| #3 | 0 | 19,2 ± 1,0* |
| | 2 | 1 5,6 ± 1,3* |
| | 4 | 14,5 ± 1,1* |
| | 24 | 11,3 ± 0,8* |
| #4 | 0 | 17,1 ± 1,3* |
| | 2 | 15,3 ± 1,1* |
| | 4 | 13,6 ± 0,9* |
| | 24 | 11,5 ± 0,8* |
| #5 | 0 | 20,2 ± 1,0* |
| | 2 | 15,3 ± 1,2* |
| | 4 | 12,0 ± 1,0* |
| | 24 | 9,1 ± 0,8 |

The results demonstrated that a short exposure to a discontinuous light treatment don't affect viability however generates a clear motility increase in frozen seminal doses in some of the treatments tested.

According to the proposed method, once the treatment of the sperm cells has been performed according to the principles of the invention, it has been verified that the percentage of motile spermatozoa and their level of motility improved with respect to their prior baseline level, as has their stress resistance capacity.

Since all the factors which improve the viability and motility of motile spermatozoa are a key factor in a successful ovule fertilization process, this enhancement of cell viability and vigor will increase fertilization probabilities.

With reference now to Figs. 1 and 2, these figures illustrate an embodiment of an apparatus for implementing the proposed method for improving the quality of mammalian sperm, preferably ruminants and equines sperm cells, which are diluted in a diluent with cryo-preservatives solutes, included in a container 2 such as a straw and kept in a frozen state therein. The walls of the container are transparent thereby allowing irradiation of light there through.

The apparatus of Figs. 1 and 2 comprises non-coherent red light illumination means 3, such as a plurality of LEDs, which are supported on a structure 4 and are configured and arranged for irradiating non-coherent red light to said diluted and frozen ruminants or equines sperm cells included in the container 2. Besides, a support 1 for supporting the container 2 is also included in the apparatus, as well as a casing for internally housing said support 1, the container 2 and the non-coherent red light illumination means 3.

The apparatus also comprises control means configured for controlling said illumination means 3 for performing said irradiation according to a discontinuous pattern including at least one sequence of two periods of sperm cell irradiation of a specific duration which are separated by an intermediate period of darkness of a specific duration. Each of the specific periods of irradiation and darkness can have duration between 1 and 5 minutes. Furthermore, the mentioned periods of exposure and darkness can have the same or different duration.

According to an embodiment, in this case not illustrated in the figures, overheating control means are also included in the apparatus. This overheating control means are arranged and configured for ventilate the elements housed inside said casing, so preventing temperatures greater than a greater threshold, for instance 40ºC, inside the casing, and therefore preventing the sperm cells overheating.

The scope of protection of the invention is defined by the following claims.

### References:

[1] Yeste, M., Codony, F., Estrada, E., Lleonart, M., Balasch, S., Peña, A., Rodríguez-Gil, J. E. (2016). Specific LED-based red light photo-stimulation procedures improve overall sperm function and reproductive performance of boar ejaculates. Scientific Reports, 6, 22569. http://doi.org/10.1038/srep22569.
[2] Pérez-Cerezales, S., Boryshpolets, S., Afanzar, O., Brandis, A., Nevo, R., Kiss, V., & Eisenbach, M. (2015). Involvement of opsins in mammalian sperm thermotaxis. Scientific Reports, 5, 16146. http://doi.org/10.1038/srep16146.
[3] Tafur, J., & Mills, P. J. (2008). Low-Intensity Light Therapy: Exploring the Role of Redox Mechanisms. Photomedicine and Laser Surgery, 26(4), 323-328. http://doi.org/10.1089/pho.2007.2184.
[4] Muiño, R., Tamargo, C., Hidalgo, C.O. & Peña, A.I.(2008) Identification of sperm subpopulations with defined motility characteristics in ejaculates from Holstein bulls: effects of cryopreservation and between-bull variation. Anim. Reprod. Sci. 109, 27-39
[5] Garner DL, Johnson LA (1995) Viability assessment of mammalian sperm using SYBR-14 and propidium iodide. Biol. Reprod. 53: 276-284.
[6] Prieto-Martínez, N., et al. (2016) Aquaglyceroporins 3 and 7 in bull sperm: identification, localisation and their relationship with sperm cryotolerance. Reprod. Fertil. Dev.http://dx.doi.org/10.1071/RD16077.

## Claims

1. A method for improving the quality of mammalian sperm, said method comprising irradiating sperm cells with non-coherent red light in a discontinuous manner according to a discontinuous pattern including at least one sequence of two periods of sperm cell irradiation of a specific duration which are separated by an intermediate period of darkness of a specific duration, said sperm cells being included in a container having walls transparent to the wavelength of said irradiated red light,
**characterized in that**:
- the sperm cells are diluted in a diluent with cryo-preservatives solutes before being included in said container and kept in said container in a frozen state; and
- said discontinuous pattern of non-coherent red light comprise a duration between 3 and 15 minutes including said two periods of irradiation and of the intermediate period of darkness.

2. A method according to claim 1, wherein the duration of said two periods of irradiation and of the intermediate period of darkness is the same.

3. The method according to any of previous claims, wherein each of said specific periods of irradiation and darkness has a duration between 1 and 5 minutes.

4. The method according to claim 1 or 2, wherein each of said specific periods of irradiation and darkness has a duration of 2 minutes.

5. The method according to the claim 1, wherein said periods of irradiation and darkness are of different duration.

6. The method according to any of the previous claims, wherein said red light has a wavelength between 620 and 640 nm.

7. The method according to any of the previous claims, wherein said diluted sperm cells have a volume comprised in a range between 120µl to 250µl, and said container being a straw.

8. The method of claim 1, comprising keeping the diluted but frozen sperm cells at least until a period of time immediately before the application of said discontinuous pattern of non-coherent red light thereon and/or during said application.

9. The method according to claim 1 or 8, comprising incubating in a heat controlled medium at substantially near to animal physiological temperature up to 35-40ºC the sperm cells after being subjected to said discontinuous pattern of non-coherent red light.

10. The method according to claim 9, wherein said incubation is performed for different periods of time, after each of which the method comprises performing analytical controls on the sperm cells for determining at least a state of functional viability and motility of the spermatozoa.

11. The method according to any one of the preceding claims, where the sperm cells are mammalian sperm cells, including at least ruminant and/or equine sperm cells.

12. An apparatus for improving the quality of mammalian sperm, the apparatus comprising:
- non-coherent red light illumination means (3) configured and arranged for irradiating sperm cells with non-coherent red light;
- control means configured for controlling said illumination means (3) for performing said irradiation according to a discontinuous pattern including at least one sequence of two periods of sperm cell irradiation of a specific duration which are separated by an intermediate period of darkness of a specific duration; and
- a support (1) supporting at least one container (2) for containing said sperm cells, and the walls of which are transparent to the wavelength of said irradiated red light; and
- a casing for internally housing said support (1), the at least one container (2) and the non-coherent red light illumination means (3),
**characterized in that** the control means are configured to perform said discontinuous pattern of non-coherent red light during a period comprised between 3 and 15 minutes, wherein the sperm cells being diluted in a diluent with cryo-preservatives solutes and being kept in the container (2) in a frozen state.

13. The apparatus of claim 12, wherein said container (2) comprises a straw.

14. The apparatus of claim 12 or 13, comprising overheating control means arranged and configured for ventilate the elements housed inside said casing (1).
